# FASCICULE DE BREVET EUROPEEN

(11) **EP 2 709 569 B1**
(45) Date de publication et mention de la délivrance du brevet: **13.05.2015**
(21) Numéro de dépôt: 12724679.1
(22) Date de dépôt: 23.04.2012
(51) Int. Cl.: A61F 5/055

(54) **COLLIER CERVICAL**
HALSKRAUSE
CERVICAL COLLAR

(30) Priorité: 16.05.2011 FR 1154243
(43) Date de publication de la demande: 26.03.2014
(73) Titulaire: Gibaud, 42000 Saint Etienne (FR)
(72) Inventeur: GIRARD, Frédéric, 75009 Paris (FR); DUPORT, Guillaume, 42000 Saint Etienne (FR)
(74) Mandataire: Delorme, Nicolas
(86) Numéro de dépôt international: PCT/FR2012/050881
(87) Numéro de publication internationale: WO 2012/156607

(56) Documents cités:
- FR-A1- 2 582 936
- US-A- 3 055 358
- US-A- 3 850 164
- US-A- 5 904 662

## Description

La présente invention concerne un collier cervical.

Il est connu de réaliser des colliers cervicaux comprenant principalement un corps en mousse destiné à enserrer le cou d'un patient afin de restreindre les mouvements de sa tête par rapport au reste de son corps et maintenir en position ses vertèbres cervicales. Ces colliers cervicaux présentent un intérêt pour tout patient souffrant de traumatismes ou de rhumatismes affectant les vertèbres cervicales.

Les colliers cervicaux existants donnent généralement satisfaction dans leur fonction de maintien des vertèbres cervicales. Cependant, ils offrent parfois un confort insuffisant pour le patient. En particulier, les colliers cervicaux connus peuvent provoquer la transpiration du patient et n'offrent généralement pas la possibilité de prévenir cette transpiration ou de l'évacuer.

Aussi la présente invention a pour but de résoudre tout ou partie des inconvénients mentionnés ci-dessus.

A cet effet, la présente invention a pour objet un collier cervical comprenant un corps en mousse destiné à entourer le cou d'un patient, une ouverture débouchante étant ménagée dans le corps. De plus, selon une définition générale de l'invention, le collier cervical comprend un insert en mousse placé dans l'ouverture dudit corps, ledit insert présentant une face interne, destinée à être tournée vers le cou d'un patient, et une face externe, ledit insert comprenant une pluralité de perforations, chaque perforation de l'insert débouchant de part et d'autre de l'insert, d'un côté sur la face interne et de l'autre côté sur la face externe, afin de favoriser l'évacuation de la transpiration du patient et d'améliorer la respirabilité du collier cervical.

Ainsi, le collier cervical selon l'invention muni de son insert en mousse perforée permet d'améliorer la respirabilité du collier cervical et de prévenir et évacuer aisément la transpiration du patient.

Cet avantage n'est pas obtenu au détriment de la rigidité du collier, qui est essentielle pour que le collier assure un maintien satisfaisant de la zone cervicale du patient. En effet, l'ouverture étant localisée dans le corps, il ne s'ensuit pas une trop grande perte de rigidité du corps, qui continue à assurer cette fonction. De plus, l'insert apporte également une certaine rigidité.

Il est à noter que, de préférence, l'insert comble sensiblement l'ouverture pratiquée dans le corps. En d'autres termes, la périphérie de l'insert est adjacente et en contact avec la périphérie de l'ouverture. De plus, les faces intérieure - tournée vers le cou du patient - et extérieure de l'insert sont de préférence situées dans la continuité des faces intérieure et extérieure, respectivement, du corps. Le collier ne présente ainsi sensiblement aucune surépaisseur ou aucune dépression au niveau de l'insert.

Un autre avantage de l'invention réside dans le fait que la périphérie du corps conserve son aspect lisse puisque les perforations sont localisées dans l'insert et non dans le corps, de préférence. Ainsi, il n'existe pas au niveau de la périphérie du corps de cavités ou d'échancrures formées par ces perforations. En conséquence, d'une part, il n'existe pas d'amorce de rupture qui pourrait conduire à la dégradation plus ou moins rapide du collier et, d'autre part, l'aspect esthétique du collier est préservé.

Par « ouverture débouchante », on entend que cette ouverture traverse le corps depuis sa face intérieure - tournée vers le cou du patient - jusqu'à se face extérieur. Toutefois, avantageusement, l'ouverture pratiquée dans le corps ne débouche pas sur les faces supérieure, inférieure ou latérales du corps.

Selon une autre caractéristique du collier cervical selon l'invention, celui-ci peut comprendre une doublure à l'intérieur de laquelle sont disposés le corps en mousse et l'insert.

Cette doublure contribue au maintien en place de l'insert à l'intérieur du corps en mousse. Cette doublure, qui peut être en tissu tricoté extensible, permet également de gainer le corps en mousse dans lequel est placé l'insert. Enfin, cette doublure, dont une partie est destinée à venir au contact du cou du patient, améliore le confort de ce dernier.

Par exemple, l'insert peut être en mousse de densité égale ou supérieure à celle du corps. En choisissant pour l'insert une mousse de densité supérieure à celle de la mousse constituant le corps, on peut compenser la perte de rigidité du collier due à la présence de perforations dans l'insert. En conséquence, la rigidité du corps et celle de l'insert peuvent être comparables.

On peut prévoir que l'ouverture s'étende selon une direction sensiblement longitudinale, le long du corps.

En outre, l'ouverture peut être symétrique par rapport à un plan séparant le corps en deux parties égales, ledit plan étant sensiblement parallèle au plan sagittal d'un patient. Cette caractéristique permet de répartir équitablement la propriété de respirabilité du collier cervical selon l'invention.

L'ouverture peut être unique. De ce fait, le collier est plus simple et plus rapide à fabriquer et à monter.

L'insert peut présenter sur son pourtour au moins une extension en contact avec la paroi interne de l'ouverture. De manière avantageuse, cela permet d'augmenter le frottement entre le corps et l'insert pour éviter que l'insert ne se déplace à l'intérieur du corps jusqu'à s'en déloger.

Chaque extension peut être partie intégrante de l'insert.

On peut par ailleurs prévoir que l'insert comprenne des charges céramiques. Cette caractéristique permet avantageusement de réfléchir le rayonnement infrarouge émis par le patient en vue de compenser la perte d'isolation thermique due aux perforations comportées par l'insert.

Les perforations de l'insert sont par exemple situées en périphérie de l'insert, ce qui permet de limiter l'impact sur la rigidité du collier cervical.

On décrit à présent, à titre d'exemples non limitatifs, plusieurs modes de réalisation possibles de l'invention, en référence aux figures annexées :
La figure 1 est une vue en perspective d'un collier cervical selon un premier mode de réalisation de l'invention, le collier comprenant un corps pourvu d'une ouverture et un insert monté dans cette ouverture, ainsi qu'une doublure enveloppante qui n'est que partiellement représentée ;
La figure 2 est une vue en perspective du corps du collier cervical de la figure 1 ;
La figure 3 est une vue en perspective de l'insert du collier cervical de la figure 1 ;
La figure 4 est une vue schématique, à plat, d'un collier cervical selon un deuxième mode de réalisation de l'invention ;
La figure 5 est une vue schématique, à plat, du corps et de l'insert du collier cervical de la figure 4, le corps et l'insert étant séparés.

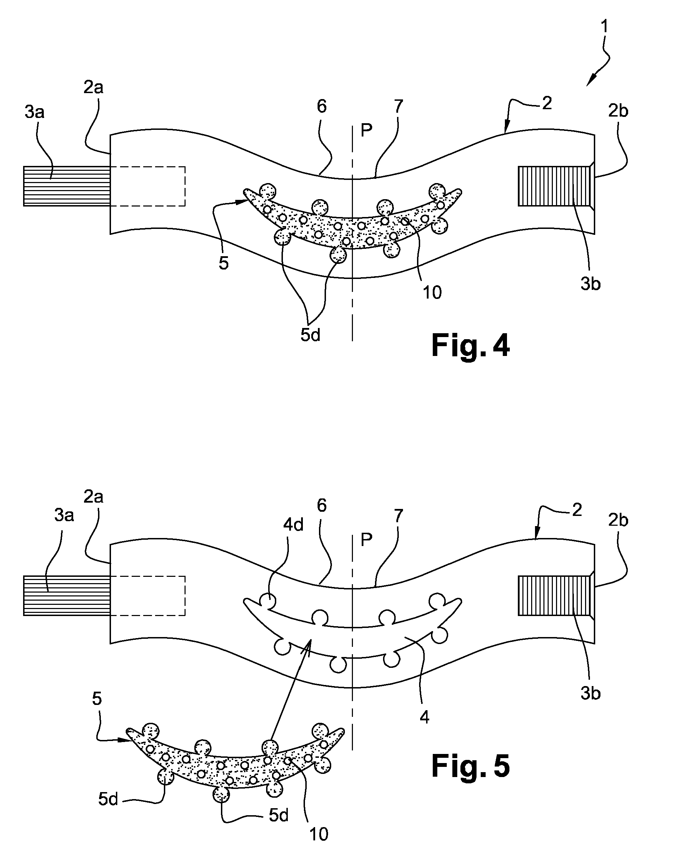

Comme illustré sur les figures, le collier 1 cervical selon l'invention comprend un corps 2 présentant une première extrémité 2a et une deuxième extrémité 2b. Le corps 2 peut être réalisé en mousse, par exemple une mousse de polyester présentant une densité de l'ordre de 35 kg/m³. Le corps 2 s'étend sensiblement longitudinalement de sa première extrémité 2a vers sa seconde extrémité 2b, et peut posséder une forme ergonomique, par exemple définissant sensiblement une vague (comme dans la réalisation des figures 4 et 5). Le corps 2 présente de préférence un plan de symétrie P le partageant en deux parties 6, 7 symétriques par rapport à ce plan. Lorsque le collier 1 est porté, le plan P est sensiblement parallèle au plan sagittal du patient.

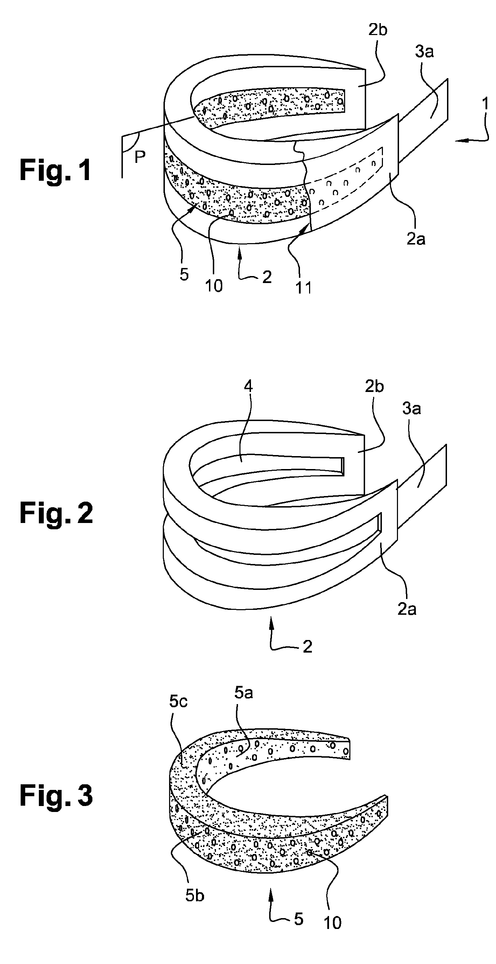

Le corps 2 comprend des moyens d'attache complémentaires 3a, 3b, par exemple du type à boucles et crochets. Les moyens d'attache complémentaires 3a, 3b permettent la fermeture du collier 1 cervical. Comme cela est visible sur la figure 1 et sur la figure 4, les moyens d'attache complémentaires 3a, 3b sont respectivement placés au niveau de la première extrémité 2a et de la deuxième extrémité 2b du collier 1 cervical.

Le corps 2 présente une ouverture 4. L'ouverture 4 est débouchante, c'est-à-dire qu'elle est pratiquée dans l'épaisseur du corps 2, qu'elle traverse, et débouche du côté intérieur et du côté extérieur du corps 2. L'ouverture 4 peut être localisée dans la partie centrale du corps 2, comme cela est représenté sur les différentes figures, c'est-à-dire ne pas déboucher dans les côtés supérieur, inférieur ou latéraux du corps 2. Avantageusement, l'ouverture 4 s'étend de manière longitudinale le long du corps 2, de part et d'autre du plan P. Elle peut par exemple présenter une forme arquée suivant les bords supérieur et inférieur du corps 2, comme sur les figures 4 et 5.

Dans les réalisations représentées, l'ouverture 4 est unique.

Le corps 2 est par exemple obtenu par découpe d'un bloc de mousse grâce à un outil de coupe. Il peut s'agir d'un outil de type emporte-pièce qui permet simultanément de découper le contour du corps 2 et de l'ouverture 4.

A l'intérieur de l'ouverture 4 est placé un insert 5, comme cela est visible sur la figure 1 notamment. L'insert 5 présente de préférence une forme complémentaire de celle de l'ouverture 4, afin de pouvoir combler sensiblement toute l'ouverture 4. Dans la réalisation représentée sur les figures 4 et 5, l'insert 5 peut présenter une forme arquée qui suit la forme ergonomique du corps 2. En pratique, l'insert 5 peut être monté à force dans l'ouverture 4 du corps 2.

Comme cela est représenté à la figure 3, l'insert 5 comprend une face interne 5a, destinée à être tournée vers le cou du patient, une face externe 5b, qui est opposée à la face interne 5a et n'entre en contact ni avec le corps 2 ni avec le patient, ainsi qu'un pourtour 5c formé par la surface de l'insert 5 comprise entre la face interne 5a et la face externe 5b.

L'insert 5 peut être réalisé en mousse. Il peut s'agir d'une mousse différente de celle du corps 2, par exemple une mousse de polyester, de densité égale ou supérieure à celle du corps 2. Par exemple, la densité de la mousse du corps 2 est de l'ordre de 35 kg/m³ ; celle de la mousse de l'insert 5 peut être comprise entre 35 et 50 kg/m³, voire entre 38 et 45 kg/m³.

L'insert 5 comporte des perforations 10. Celles-ci traversent l'insert 5 de part en part. Ainsi, les perforations 10 de l'insert 5 débouchent sur la face interne 5a et la face externe 5b, de sorte que le cou du patient se trouve, via ces perforations 10, en contact direct avec l'air. Les perforations 10 de l'insert 5 peuvent être obtenues par exemple par piquage, lorsque la mousse dans laquelle est découpé l'insert 5 est écrasée contre un cylindre dont la surface est revêtue de saillies ou motifs. Les perforations 10 de l'insert 5 peuvent également être obtenues par des moyens mécaniques (outils de coupe), thermiques (découpe ultrasonique,...), découpage laser ou même chimiques (dissolution ponctuelle par solvant).

Par exemple, l'insert 5 peut posséder une densité de trous de l'ordre de 10% et être réalisé en une mousse de densité de l'ordre de 38 à 40 kg/m³. En variante, l'insert 5 peut posséder une densité de trous de l'ordre de 20% et être réalisé en une mousse de densité de l'ordre de 42 à 450 kg/m³.

Dans le premier mode de réalisation illustré sur les figures 1 à 3, le pourtour 5c de l'insert 5 forme des faces sensiblement planes.

Selon le deuxième mode de réalisation représenté sur les figures 4 et 5, l'insert 5 peut comporter des extensions 5d qui sont situées sur le pourtour 5c de l'insert 5. Les extensions 5d s'étendent vers l'extérieur de l'insert 5, entre un plan contenant la surface interne 5a de l'insert 5, et un plan contenant la face externe 5b de l'insert 5.

L'ouverture 4 présente alors une forme complémentaire de celle de l'insert 5 avec ses extensions 5d, et comprend donc des cavités 4d. Ainsi, les extensions 5d sont en contact avec la paroi interne de l'ouverture 4 lorsque l'insert 5 est inséré dans l'ouverture 4, les extensions 5d étant logées dans les cavités 4d.

La mousse de l'insert 5 peut comporter des charges céramiques permettant de réfléchir le rayonnement infrarouge émis par le patient. Plus généralement, on peut prévoir dans l'insert 5 des moyens thermiquement isolants améliorant l'effet antalgique du collier 1 par le maintien d'une certaine chaleur localement sur le patient.

Il peut également être prévu que l'insert puisse dégager des huiles essentielles visant à améliorer le confort et/ou le caractère antalgique du collier 1. Par ailleurs, on pourrait prévoir de réaliser l'insert 5 dans une couleur différente de celle du corps 2 pour mettre en évidence, pour le patient, le fait que le collier 1 possède un tel insert 5 lui conférant des avantages par rapport aux colliers cervicaux conventionnels.

Une doublure 11 en tissu, qui peut être tricotée, recouvre le corps 2 et l'insert 5 en les enveloppant, pour davantage de confort et pour une meilleure absorption de la transpiration. Cette doublure 11 est représentée partiellement sur la figure 1.

Avec ce collier cervical, l'invention apporte donc une amélioration importante par rapport à l'art antérieur, en permettant d'améliorer la respirabilité du collier sans dégrader son aspect ou ses propriétés de maintien.

## Revendications

1. Collier cervical comprenant un corps (2) en mousse destiné à entourer le cou d'un patient, une ouverture (4) débouchante étant ménagée dans le corps (2), **caractérisé en ce que** le collier (1) cervical comprend un insert (5) en mousse placé dans l'ouverture (4) dudit corps (2), ledit insert (5) présentant une face interne (5a), destinée à être tournée vers le cou d'un patient, et une face externe (5b), ledit insert (5) comprenant une pluralité de perforations (10), chaque perforation de l'insert (5) débouchant de part et d'autre de l'insert (5), d'un côté sur la face interne (5a) et de l'autre côté sur la face externe (5b), afin de favoriser l'évacuation de la transpiration du patient et d'améliorer la respirabilité du collier (1) cervical.

2. Collier cervical selon la revendication 1, **caractérisé en ce que** l'insert (5) est en mousse de densité égale ou supérieure à celle du corps (2).

3. Collier cervical selon l'une des revendications 1 à 2, **caractérisé en ce que** l'ouverture (4) s'étend selon une direction sensiblement longitudinale, le long du corps (2).

4. Collier cervical selon l'une des revendications 1 à 3, **caractérisé en ce que** l'ouverture (4) est symétrique par rapport à un plan (P) séparant le corps (2) en deux parties (6, 7) égales, ledit plan (P) étant sensiblement parallèle au plan sagittal d'un patient.

5. Collier cervical selon l'une des revendications 1 à 4, **caractérisé en ce que** l'ouverture (4) est unique.

6. Collier cervical selon l'une des revendications 1 à 5, **caractérisé en ce que** l'insert (5) présente sur son pourtour (5c) au moins une extension (5d) en contact avec la paroi interne de l'ouverture (4).

7. Collier cervical selon l'une des revendications 1 à 6, **caractérisé en ce que** chaque extension (5d) est partie intégrante de l'insert (5).

8. Collier cervical selon l'une des revendications 1 à 7, **caractérisé en ce que** l'insert (5) comprend des charges céramiques.

9. Collier cervical selon l'une des revendications 1 à 8, **caractérisé en ce que** les perforations (10) de l'insert (5) sont situées en périphérie de l'insert (5).

10. Collier cervical selon l'une des revendications 1 à 9, **caractérisé en ce qu'**il comprend une doublure (11) à l'intérieur de laquelle sont disposés le corps (2) en mousse et l'insert (5).

## Patentansprüche

1. Halskrause, umfassend einen Körper (2) aus Schaum, der ausgelegt ist, um den Hals eines Patienten zu umgeben, eine Mündungsöffnung (4), die im Körper (2) angeordnet ist, **dadurch gekennzeichnet, dass** die Halskrause (1) einen Einsatz (5) aus Schaum umfasst, der in der Öffnung (4) der Körpers (2) angeordnet ist, wobei der Einsatz (5) eine innere Fläche (5a) aufweist, die ausgelegt ist, um gegen den Hals eines Patienten gedreht zu sein, und eine äußere Fläche (5b), wobei der Einsatz (5) eine Vielzahl von Perforationen (10) umfasst, wobei jede Perforation des Einsatzes (5) auf beiden Seiten des Einsatzes (5) mündet, auf der einen Seite auf der inneren Fläche (5a) und auf der anderen Seite auf der äußeren Fläche (5b), um die Ableitung des Schweißes des Patienten zu erleichtern und die Atmungsfähigkeit der Halskrause (1) zu verbessern.

2. Halskrause nach Anspruch 1, **dadurch gekennzeichnet, dass** der Einsatz (5) aus Schaum mit einer Dichte besteht, die gleich oder höher als diejenige des Körpers (2) ist.

3. Halskrause nach einem der Ansprüche 1 bis 2, **dadurch gekennzeichnet, dass** sich die Öffnung (4) gemäß einer im Wesentlichen Längsrichtung entlang dem Körper (2) erstreckt.

4. Halskrause nach einem der Ansprüche 1 bis 3, **dadurch gekennzeichnet, dass** die Öffnung (4) symmetrisch mit Bezug auf eine Ebene (P) ist, die den Körper (2) in zwei gleiche Teile (6, 7) trennt, wobei die Ebene (P) im Wesentlichen parallel zur sagittalen Ebene eines Patienten ist.

5. Halskrause nach einem der Ansprüche 1 bis 4, **dadurch gekennzeichnet, dass** die Öffnung (4) einzigartig ist.

6. Halskrause nach einem der Ansprüche 1 bis 5, **dadurch gekennzeichnet, dass** der Einsatz (5) auf seinem Umfang (5c) mindestens eine Erweiterung (5d) aufweist, die in Kontakt mit der inneren Wand der Öffnung (4) steht.

7. Halskrause nach einem der Ansprüche 1 bis 6, **dadurch gekennzeichnet, dass** jede Erweiterung (5d) ein integraler Bestandteil des Einsatzes (5) ist.

8. Halskrause nach einem der Ansprüche 1 bis 7, **dadurch gekennzeichnet, dass** der Einsatz (5) keramische Füllungen umfasst.

9. Halskrause nach einem der Ansprüche 1 bis 8, **dadurch gekennzeichnet, dass** sich die Perforationen (10) des Einsatzes (5) am der Peripherie des Einsatzes (5) befinden.

10. Halskrause nach einem der Ansprüche 1 bis 9, **dadurch gekennzeichnet, dass** sie ein Futter (11) umfasst, in dessen Inneren der Körper (2) aus Schaum und der Einsatz (5) angeordnet sind.

## Claims

1. A cervical collar comprising a body (2) made of foam intended to surround the neck of a patient, a through opening (4) being formed in the body (2), **characterized in that** the cervical collar (1) comprises an insert (5) made of foam placed in the opening (4) of said body (2), said insert (5) having an inner face (5a), intended to be turned towards the neck of a patient, and an outer face (5b), said insert (5) comprising a plurality of perforations (10), each perforation of the insert (5) opening on either side of the insert (5), on one side on the inner face (5a) and on the other side on the outer face (5b) in order to promote the evacuation of perspiration of the patient and improve the breathability of the cervical collar (1).

2. The cervical collar according to claim 1, **characterized in that** the insert (5) is made of foam with a density equal to or higher than that of the body (2).

3. The cervical collar according to any of claims 1 to 2, **characterized in that** the opening (4) extends along a substantially longitudinal direction, along the body (2).

4. The cervical collar according to any of claims 1 to 3, **characterized in that** the opening (4) is symmetrical with respect to a plane (P) separating the body (2) into two equal parts (6, 7), said plane (P) being substantially parallel to the sagittal plane of a patient.

5. The cervical collar according to any of claims 1 to 4, **characterized in that** the opening (4) is unique.

6. The cervical collar according to any of claims 1 to 5, **characterized in that** the insert (5) has on its rim (5c) at least one extension (5d) in contact with the inner wall of the opening (4).

7. The cervical collar according to any of claims 1 to 6, **characterized in that** each extension (5d) is an integral part of the insert (5).

8. The cervical collar according to any of claims 1 to 7, **characterized in that** the insert (5) comprises ceramic fillers.

9. The cervical collar according to any of claims 1 to 8, **characterized in that** the perforations (10) of the insert (5) are located on the periphery of the insert (5).

10. The cervical collar according to any of claims 1 to 9, **characterized in that** it comprises a lining (11) within which the body (2) made of foam and the insert (5) are disposed.
